# FASCICULE DE BREVET EUROPEEN

(11) **EP 1 149 101 B1**
(45) Date de publication et mention de la délivrance du brevet: **09.04.2003**
(21) Numéro de dépôt: 00900636.2
(22) Date de dépôt: 21.01.2000
(51) Int. Cl.: C07D 487/04, A61K 31/50, A61P 25/02, A61P 25/28

(54) **DERIVES DE 4-OXO-3,5-DIHYDRO-4H-PYRIDAZINO [4,5-B] INDOLE-1-CARBOXAMIDE, LEUR PREPARATION ET LEUR APPLICATION EN THERAPEUTIQUE**
4-OXO-3,5-DIHYDRO-4H-PYRIDAZINO[4,5-B]INDOL-1-CARBOXAMID-DERIVATE, IHRE HERSTELLUNG UND THERAPEUTISCHE VERWENDUNG
4-OXO-3,5-DIHYDRO-4$i(H)-PYRIDAZINO 4,5-$i(b)]INDOLE-1-CARBOXAMIDE DERIVATIVES, PREPARATION AND THERAPEUTIC USE

(30) Priorité: 26.01.1999 FR 9900805
(43) Date de publication de la demande: 31.10.2001
(73) Titulaire: SANOFI-SYNTHELABO, 75013 Paris (FR)
(72) Inventeur: MARGUET, Frank, F-91370 Verrières le Buisson (FR); FROISSANT, Jacques, F-41160 Morée (FR); BARTSCH-LI, Régine, F-92260 Fontenay aux Roses (FR); MARABOUT, Benoît, F-91380 Chilly Mazarin (FR); SEVRIN, Mireille, F-75014 Paris (FR)
(74) Mandataire: Ludwig, Jacques
(86) Numéro de dépôt international: FR0000134
(87) Numéro de publication internationale: WO00044751

(56) Documents cités:
- WO-A-98/15552
- WO-A-99/06406

## Description

La présente invention a pour objet des composés de formule générale (I) 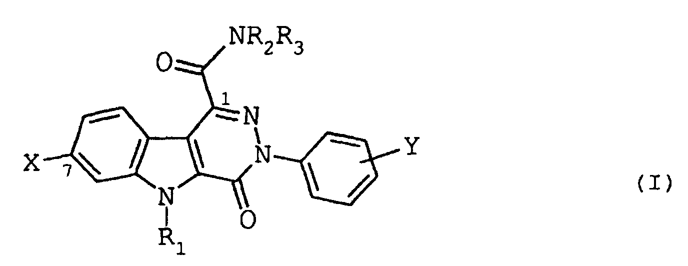 dans laquelle
X représente un atome d'hydrogène ou d'halogène,
Y représente un ou plusieurs atomes ou groupes choisis parmi l'hydrogène, les halogènes et les groupes méthyle, méthoxy et nitro,
R₁ représente un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle ou morpholinyle.

Les composés préférés sont ceux dans la formule générale desquels X représente un atome d'halogène, Y représente un atome d'hydrogène ou un atome d'halogène, R₁ représente un groupe méthyle, R₂ représente un atome d'hydrogène ou un groupe méthyle, R₃ représente un groupe méthyle, ou bien R₂ et R₃ forment, avec l'atome d'azote qui les porte, un cycle pyrrolidinyle.

Des composés de structure analogue à celle des composés de l'invention sont décrits dans la demande internationale WO 99 06406

Les composés de formule générale (I) peuvent être préparés par des procédés illustres par le schéma qui suit.

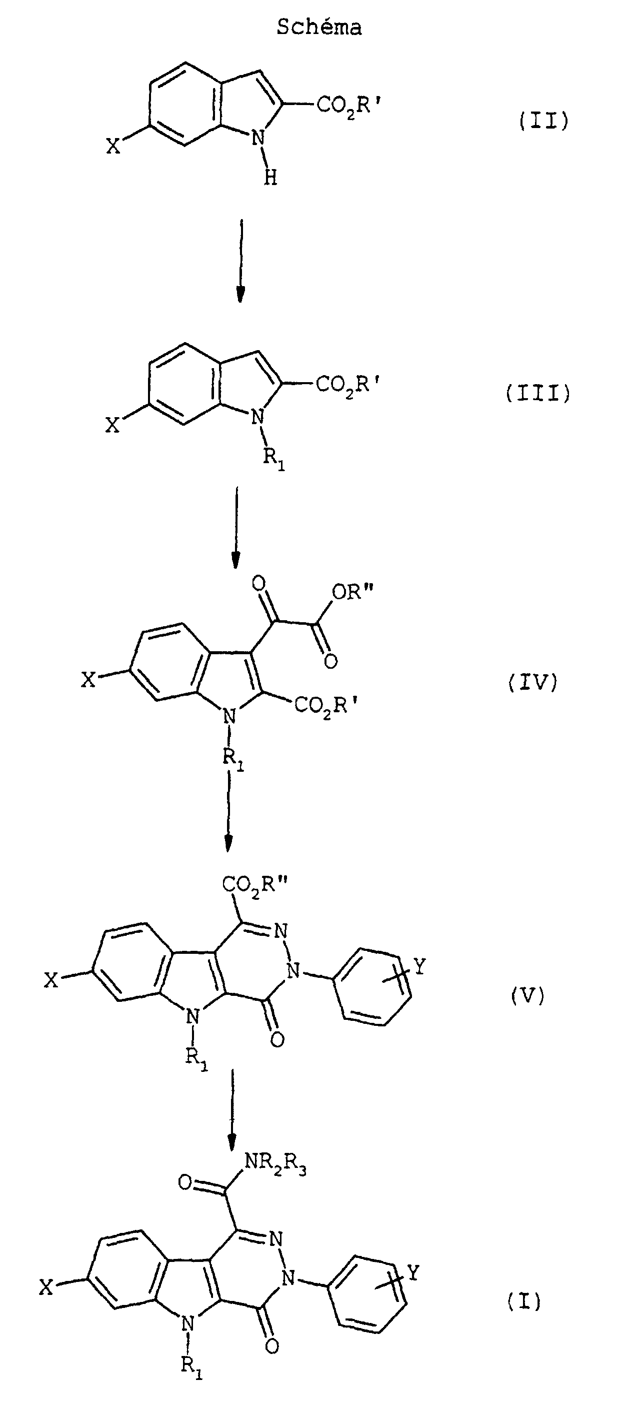

Selon ce schéma on soumet un composé de formule générale (II), dans laquelle X est tel que défini ci-dessus et R' représente un groupe (C₁-C₄)alkyle, à une réaction d'alkylation avec un halogénure de formule générale HalR₁ dans laquelle R₁ est tel que défini ci-dessus pour aboutir à un composé formule générale (III). On fait réagir ensuite ce composé avec un chlorooxoacétate de formule générale ClCOCO₂R", dans laquelle R" représente un groupe (C₁-C₄)-alkyle, dans un solvant aprotique polaire tel que le dichloroéthane, à température ambiante, en présence d'un acide de Lewis, par exemple le tétrachlorure de titane, pour obtenir le diester de formule générale (IV).
On traite ensuite ce dernier dans l'acide acétique, d'abord à température ambiante puis à la température de reflux, avec une phénylhydrazine éventuellement substituée par un groupe Y tel que défini ci-dessus, pour obtenir un ester de formule générale (V).
On transforme finalement cet ester en amide secondaire ou tertiaire de formule générale (I) par action d'une amine de formule générale HNR₂R₃, dans laquelle R₂ et R₃ sont tels que définis ci-dessus, par exemple en présence d'un dérivé trialkylaluminium dans un solvant tel que le toluene.

Les composés de départ de formule générale (II) sont connus de l'homme du métier. A titre indicatif on mentionnera la formation de dérivés d'acide 6-chloroindole-2-carboxylique selon Rydon *et al. J. Chem. Soc.* (1955) 3499.

Les exemples qui vont suivre illustrent la préparation de quelques composés selon l'invention. Les microanalyses élémentaires, et les spectres I.R. et R.M.N. confirment les structures des composés obtenus.
Les numéros indiqués entre parenthèses dans les titres des exemples correspondent à ceux de la lère colonne du tableau donné plus loin.

Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°8).

### 1-[[7-chloro-3-(3-chlorophényl)-5-méthyl-4-oxo-3,5-dihydro-4H-pyridazino[4,5-b]indol-1-yl]carbonyl]pyrrolidine

### 1.1. 6-Chloro-1-méthyl-1H-indole-2-carboxylate d'éthyle.

On agite pendant 2 h à température ambiante une suspension de 1,8 g (45 mmoles) d'hydrure de sodium à 60% (préalablement lavé à l'éther de pétrole) et de 8,0 g (35,8 mmoles) de 6-chloro-1*H*-indole-2-carboxylate d'éthyle dans 80 ml de *N,N*-diméthylformamide, on ajoute ensuite 2,8 ml (45 mmoles) de iodométhane et on agite le mélange à température ambiante pendant 4 H.
On ajoute 5 ml d'éthanol absolu et on évapore le solvant sous pression réduite. On reprend le résidu par de l'eau et on extrait le mélange par du dichlorométhane, on sèche la phase organique, on filtre, on évapore le solvant sous pression réduite et on purifie le résidu par chromatographie sur colonne de gel de silice.
On isole 8,5 g (35,7 mmoles) d'un composé blanc cristallin. Point de fusion : 75,5-76,5°C

### 1.2. 6-chloro-2-(éthoxycarbonyl)-1-méthyl-α-oxo-1H-indole-3-acétate d'éthyle.

On refroidit à 0°C une solution de 7,1 ml (63,6 mmoles) de chlorooxoacétate d'éthyle dans 100 ml de 1,2-dichloro_ éthane. On ajoute par petites portions 7,0 ml (63,6 mmoles) de tétrachlorure de titane et on agite pendant 30 min à 0°C. On ajoute une solution de 7,4 g (35,3 mmoles) de 6-chloro-1-méthyl-*1H*-indole-2-carboxylate d'éthyle et on agite pendant 3 h à température ambiante. On verse le mélange sur de l'eau, on décante la phase organique, on la lave par de la soude diluée, on la sèche sur sulfate de sodium et on la concentre sous pression réduite pour obtenir une huile que l'on purifie par chromatographie sur colonne de silice (éluant : cyclohexane/dichlorométhane : 2/8) .
On isole 9,51 g (29,4 mmoles) de composé sous forme d'un solide blanc.
Point de fusion : 94-95°C.

### 1.3. 7-chloro-3-(3-chlorophényl)-5-méthyl-4-oxo-3,5-dihydro-4H-pyridazino[4,5-b]indole-1-carboxylate d'éthyle.

On dissout 7,0 g (39,1 mmoles) de chlorhydrate de 3-chloro_ phénylhydrazine dans 50 ml d'eau et 5 ml d'une solution de soude à 35%. On extrait à l'éther diéthylique. On sèche la phase organique sur sulfate de sodium, on la filtre et on la concentre sous pression réduite. On obtient une huile à laquelle on ajoute 100 ml d'acide acétique et 4,0 g (12,35 mmoles) de 6-chloro-2-(éthoxycarbonyl)-1-méthyl-α-oxo-*1H*-indole-3-acétate d'éthyle. On agite le mélange réactionnel pendant 30 min à température ambiante puis pendant 6 h au reflux.
On refroidit le milieu, on ajoute 100 ml d'eau, on recueille un insoluble par filtration, on le lave avec de l'eau et on le purifie par chromatographie sur colonne de silice (éluant : dichlorométhane/acétate d'éthyle : 98/2). On isole 4,59 g (11 mmoles) de composé sous forme d'un solide blanc.
Point de fusion : 235,5-237,5°C.

### 1.4. 1-[[7-chloro-3-(3-chlorophényl)-5-méthyl-4-oxo-3,5-dihydro-4H-pyridazino[4,5-b]indol-1-yl]carbonyl]_ pyrrolidine

On introduit sous argon 5 ml (10 mmoles) d'une solution de triméthylaluminium (2M dans le toluène) dans 80 ml de toluène. On refroidit la solution à 0°C, puis on ajoute, par petites portions, 0,84 ml (10 mmoles) de pyrrolidine. Après 20 min d'agitation à température ambiante, on ajoute 0,5 g (1,2 mmoles) de 7-chloro-3-(3-chlorophényl)-5-méthyl-4-oxo-3,5-dihydro-4*H*-pyridazino[4,5-*b*]indole-1-carboxylate d'éthyle et on chauffe à reflux durant 3 h.
On refroidit la solution vers 0°C et on hydrolyse le complexe métallique lentement avec de l'eau. On ajoute 100 ml de dichlorométhane et on filtre la solution. On rince le précipité avec du dichlorométhane et on ajoute au filtrat 100 ml l'eau et 10 ml d'une solution aqueuse 1M d'acide chlorhydrique. On décante la phase organique, on la lave avec de l'eau, on la sèche sur sulfate de sodium, on la concentre sous pression réduite et on purifie le résidu par chromatographie sur colonne de silice (éluant : dichlorométhane/acétate d'éthyle : 90/10).
Après recristallisation dans un mélange de dichlorométhane et d'acétate d'éthyle, on isole 0,26 g (0,59 mmoles) de composé sous forme d'un solide blanc à l'aspect cotonneux. Point de fusion : 187-187,5°C.

### Exemple 2 (Composé N°6).

### 7-chloro-3-(3-chlorophényl)-N,N, 5-triméthyl-4-oxo-3,5-dihydro-4H-pyridazino[4, 5-b] indole-1-carboxamide.

On introduit sous argon 0,815 g (10 mmoles) de chlorhydrate de diméthylamine dans 100 ml de toluène, on refroidit la solution à 0°C, puis on ajoute, par petites portions, 5 ml (10 mmoles) d'une solution de triméthylaluminium (2M dans le toluène). Après 2 h d'agitation à température ambiante on ajoute 0,8 g (1,92 mmoles) de 7-chloro-3-(3-chloro_ phényl)-5-méthyl-4-oxo-3,5-dihydro-4*H*-pyridazino[4,5-*b*]indole-1-carboxylate d'éthyle et on chauffe à reflux durant 5 h. On refroidit la solution vers 0°C et on hydrolyse le complexe métallique lentement avec de l'eau. On ajoute 100 ml de dichlorométhane et on filtre la solution. On rince le précipité avec du dichlorométhane et on ajoute au filtrat 100 ml l'eau et 10 ml d'une solution aqueuse 1M d'acide chlorhydrique. On décante la phase organique, on la lave avec de l'eau, on la sèche sur sulfate de sodium et on la concentre sous pression réduite. On purifie le résidu par chromatographie sur colonne de silice (éluant : dichlorométhane/acétate d'éthyle : 80/20).
Après recristallisation dans de l'acétate d'éthyle on isole 0,43 g (1,0 mmoles) de composé sous forme de solide blanc. Point de fusion : 212,5-213,5°C.

### Exemple 3 (Composé N°10).

### 7-chloro-N, N, 5-triméthyl-4-oxo-3-phényl-3, 5-dihydro-4H-pyridazino[4,5-b]indole-1-carboxamide.

### 3.1. 7-chloro-5-méthyl-4-oxo-3-phényl-3,5-dihydro-4H-pyridazino[4,5-b]indole-1-carboxylate d'éthyle.

On opère comme dans l'exemple 1.3. à partir de 2,0 g (18 mmoles) de phénylhydrazine et de 4,6 g (14,21 mmoles) de 6-chloro-2-(éthoxycarbonyl)-1-méthyl-α-oxo-*1H*-indole-3-acétate d'éthyle dans 100 ml d'acide acétique.
Après réaction, on refroidit le milieu, on ajoute 100 ml d'eau, on isole un insoluble par filtration et on le lave à l'eau. On le purifie par chromatographie sur silice (éluant : dichlorométhane/acétate d'éthyle : 99/1). On isole 2,98 g (7,8 mmoles) de composé sous forme d'un solide blanc.
Point de fusion :216-218,5°C.

### 3.2. 7-chloro-N,N,5-triméthyl-4-oxo-3-phényl-3,5-dihydro-4H-pyridazino[4,5-b]indole-1-carboxamide.

On opère comme dans l'exemple 2 à partir de 0,815 g (10 mmoles) de chlorhydrate de diméthylamine, de 5 ml (10 mmoles) d'une solution de triméthylaluminium (2M dans le toluène) et de 0,74 g (1,94 mmoles) de 7-chloro-5-méthyl-4-oxo-3-phényl-3,5-dihydro-4*H*-pyridazino[4,5-*b*]indole-1-carboxylate d'éthyle dans 80 ml de toluène.
Après réaction, on refroidit la solution vers 0°C et on hydrolyse le complexe métallique lentement avec de l'eau. On ajoute 100 ml de dichlorométhane et on filtre la solution. On rince le précipité avec du dichlorométhane et on ajoute au filtrat 100 ml l'eau et 10 ml d'une solution aqueuse 1M d'acide chlorhydrique. On décante la phase organique, on la lave avec de l'eau, on la sèche sur sulfate de sodium et on la concentre sous pression réduite. On purifie le résidu par chromatographie sur colonne de silice (éluant : dichlorométhane/acétate d'éthyle : 80/20). Après recristallisation dans un mélange d'acétate d'éthyle et de dichlorométhane, on isole 0,385 g (1,0 mmoles) de composé sous forme d'un solide blanc.
Point de fusion : 254-255°C

### Exemple 4 (Composé N°5).

### 7-chloro-N,N-diéthyl-3-(3-méthoxyphényl)-5-méthyl-4-oxo-3,5-dihydro-4H-pyridazino[4,5-b]indole-1-carboxamide.

### 4.1. 7-chloro-3-(3-méthoxyphényl)-5-méthyl-4-oxo-3,5-dihydro-4H-pyridazino[4,5-b]indole-1-carboxylate d'éthyle.

On opère comme dans l'exemple 1.3. à partir de 5,0 g (14,8 mmoles) de chlorhydrate de 3-méthoxyphénylhydrazine et de 4,7 g (14,5 mmoles) de 6-chloro-2-(éthoxycarbonyl)-1-méthyl-α-oxo*-1H*-indole-3-acétate d'éthyle dans 25 ml d'acide acétique.
Après réaction on refroidit le milieu, on ajoute 50 ml d'eau, on isole un insoluble par filtration et on le lave à l'eau. On le purifie par chromatographie sur colonne de silice (éluant : dichlorométhane/acétate d'éthyle : 99/1). On isole 4,89 g (11,9 mmoles) de composé sous forme d'un solide blanc à l'aspect cotonneux.
Point de fusion : 182,5-183°C.

### 4.2. 7-chloro-N,N-diéthyl-3-(3-méthoxyphényl)-5-méthyl-4-oxo-3,5-dihydro-4H-pyridazino[4,5-b]indole-1-carboxamide.

On opère comme dans l'exemple 1.4. à partir de 1,04 g (10 mmoles) de diéthylamine, de 5 ml (10 mmoles) d'une solution de triméthylaluminium (2M dans le toluène) et de 1,0 g (2,42 mmoles) de 7-chloro-3-(3-méthoxyphényl)-5-méthyl-4-oxo-3,5-dihydro-4*H*-pyridazino[4,5*-b*]indole-1-carboxylate d'éthyle dans 100 ml de toluène.
On refroidit la solution vers 0°C et on hydrolyse le complexe métallique lentement avec de l'eau. On ajoute 100 ml de dichlorométhane et on filtre la solution. On rince le précipité avec du dichlorométhane et on ajoute au filtrat 100 ml l'eau et 10 ml d'une solution aqueuse 1M d'acide chlorhydrique. On décante la phase organique, on la lave avec de l'eau, on la sèche sur sulfate de sodium et on la concentre sous pression réduite. On purifie le résidu par chromatographie sur colonne de silice (éluant : dichlorométhane/acétate d'éthyle : 95/5).
Après recristallisation dans de l'éther diéthylique on isole 0,83 g (1,89 mmoles) de composé sous forme d'un solide blanc.
Point de fusion : 207,5-208,5°C.

### Exemple 5 (Composé N°17).

### 7-chloro-3-(3-fluorophényl)-N,N, 5-triméthyl-4-oxo-3,5-dihydro-4H-pyridazino[4,5-b]indole-1-carboxamide.

### 5.1. 7-chloro-3-(3-fluorophényl)-5-méthyl-4-oxo-3,5-dihydro-4H-pyridazino[4,5-b]indole-1-carboxylate d'éthyle.

On opère comme dans l'exemple 1.3. à partir de 2,0 g (12,3 mmoles) de chlorhydrate de 3-fluorophénylhydrazine et de 1,75 g (5,4 mmoles) de 6-chloro-2-(éthoxycarbonyl)-1-méthyl-α-oxo-*1H*-indole-3-acétate d'éthyle dans 100 ml d'acide acétique.
Après réaction, on refroidit le milieu, on ajoute 50 ml d'eau, on isole un insoluble par filtration et on le lave à l'eau. On le purifie par chromatographie sur colonne de silice (éluant : dichlorométhane/acétate d'éthyle : 99/1). Après recristallisation dans de l'acétate d'éthyle, on isole 1,65 g (4,13 mmoles) de composé sous forme d'un solide blanc.
Point de fusion : 241-242°C.

### 5.2. 7-chloro-3-(3-fluorophényl)-N,N,5-triméthyl-4-oxo-3,5-dihydro-4H-pyridazino[4,5-b]indole-1-carboxamide.

On opère comme dans l'exemple 2 à partir de 0,65 g (8 mmoles) de chlorhydrate de diméthylamine, de 4 ml (8 mmoles) d'une solution de triméthylaluminium (2M dans le toluène) et de 0,65 g (1,63 mmoles) de 7-chloro-3-(3-fluorophényl)-5-méthyl-4-oxo-3,5-dihydro-4*H*-pyridazino[4,5-b]indole-1-carboxylate d'éthyle dans 60 ml de toluène. Après réaction, on refroidit la solution vers 0°C et on hydrolyse le complexe métallique lentement avec de l'eau. On ajoute 100 ml de dichlorométhane et on filtre la solution. On rince le précipité avec du dichlorométhane et on ajoute au filtrat 100 ml l'eau et 10 ml d'une solution aqueuse 1M d'acide chlorhydrique. On décante la phase organique, on la lave avec de l'eau, on la sèche sur sulfate de sodium et on la concentre sous pression réduite. On purifie le résidu par chromatographie sur colonne de silice (éluant : dichlorométhane/acétate d'éthyle : 90/10). Après recristallisation dans de l'acétate d'éthyle on isole 0,415 g (1,04 mmoles) de composé sous forme d'un solide blanc.
Point de fusion : 208-209°C.

Le tableau qui suit illustre les structures chimiques et les propriétés physiques de quelques composés selon l'invention.
Dans les colonnes "Y", "R₁" et "NR₂R₃", "Me" désigne un groupe méthyle, "Et" désigne un groupe éthyle, "Piperid" désigne un groupe pipéridinyle, "Pyrrolid" désigne un groupe pyrrolidinyle et "Morph" désigne un groupe morpholinyle.

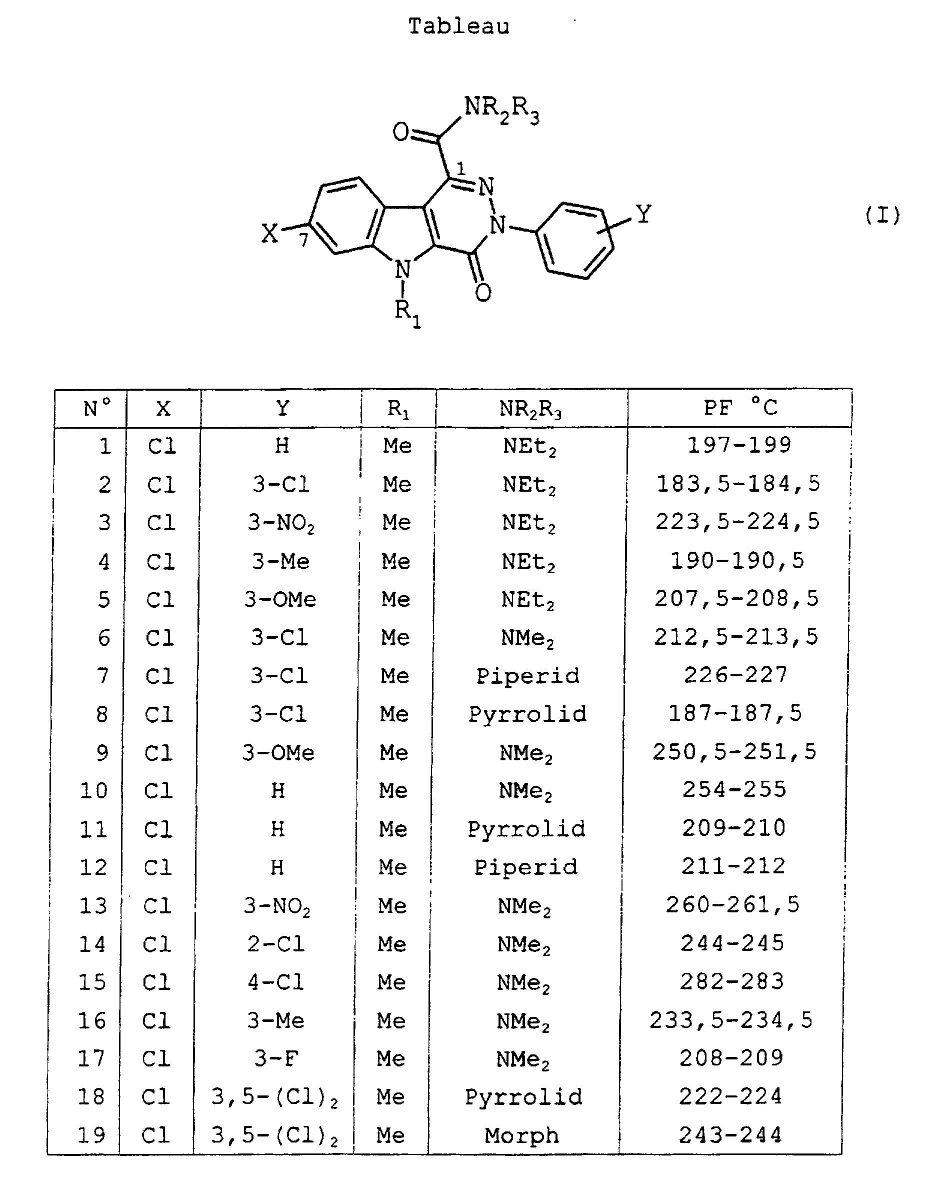

Les composés de l'invention ont été soumis à des essais pharmacologiques qui ont mis en évidence leur intérêt comme substances à activités thérapeutiques.

### Etude de la liaison [³H]Ro5-4864 aux sites benzodiazépiniques périphériques (p).

L'affinité des composés de l'invention pour les sites p (sites de liaison type périphérique pour les benzodiazépines) a été déterminée.

Les récepteurs sites p peuvent être marqués sélectivement dans des membranes de rein de rat incubées en présence de [³H]Ro5-4864. Les composés ont fait l'objet d'une étude *in vitro* quant à leur affinité pour ces récepteurs.
Les animaux utilisés sont des rats mâles Sprague Dawley (Iffa Credo) de 180 à 300 mg. Après décapitation, on prélève le rein et le tissu est homogénéisé à 4°C au moyen d'un homogénéiseur Polytron™ pendant 2 min à 6/10 de la vitesse maximale dans 35 volumes de tampon phosphate Na₂HPO₄ 50 mM à un pH ajusté à 7,5 avec du NaH₂PO₄. L'homogénat membranaire est filtré sur gaze et dilué 10 fois avec du tampon.
Le [³H]Ro5-4864 (Activité spécifique : 70-90 Ci/mmole ; New England Nuclear), à une concentration de 0,5 nM, est incubé en présence de 100 µl de l'homogénat membranaire dans un volume final de 1 ml de tampon contenant le composé à tester.
Après une incubation de 3 h à 0°C, on récupère les membranes par filtration sur filtres Whatman GF/B™ qu'on lave avec 2 fois 4,5 ml de tampon d'incubation froid (0°C). On mesure la quantité de radioactivité retenue par le filtre par scintigraphie liquide.
Pour chaque concentration de composé étudié, on détermine le pourcentage d'inhibition de la liaison du [³H]Ro5-4864, puis la concentration CI₅₀, concentration qui inhibe 50% de la liaison spécifique.
Les CI₅₀ des composés les plus actifs vont de 0,2 nM à 5 nM.

### Etude de l'activité neurotrophe.

L'activité neurotrophe est évaluée chez le rat dans le test de régénération du nerf facial lésé par mesure de la récupération fonctionnelle du reflexe palpébral selon une modification de la méthode de K. Kujawa *et al., Experimental Neurology* (1989) **105** 80-85.

La lésion du nerf facial par congélation locale entraîne une dégénérescence de la partie distale du nerf facial et une perte de la fonction du clignement de la paupière.
Les produits à étudier sont administrés par voie intrapéritonéale ou orale 2 fois par jour avec un décalage de 6 à 8 h, tous les jours pendant 10 jours (durée de l'expérience). Le premier traitement est administré 30 min avant la lésion.

### Observation des animaux

La récupération de la fonction des paupières chez les animaux lésés est observée tous les jours, une fois le matin de J0 à J5 et 2 fois (matin et soir avec 6 à 8 h de décalage) de J6 à J10, avant chaque traitement, selon un score théorique allant de 0.jusqu'à 4.

Score 0 : oeil ouvert, score 1 : oeil fermé avec un degré inférieur à la moitié de l'oeil ; score 2 : degré de fermeture compris entre 1/2 et 3/4 ; score 3 : degré de fermeture supérieur à 3/4 ; score 4 : oeil complètement fermé.
Les résultats sont exprimés par le rapport des AUC ("area under the ourve") du groupe traité et du groupe témoin.

Les rapports d'AUC des composés les plus actifs se situent entre 1,10 et 1,20.
Ces composés augmentent donc de 10 à 20% la récupération du réflexe palpébral après lésion du nerf facial.

Les résultats des essais montrent que les composés de formule générale (I) favorisent la régénération nerveuse.

Ils peuvent donc être utilisés pour la préparation de médicaments destinés à la prévention et au traitement des neuropathies périphériques de différent types, comme les neuropathies traumatiques ou ischémiques, neuropathies infectieuses, alcooliques, médicamenteuses ou génétiques, ainsi que des affections du motoneurone, telle que les amyotrophies spinales et la sclérose latérale amyotrophique. Ces médicaments trouveront également une application dans le traitement des maladies neurodégénératives du système nerveux central, soit de type aigu comme les accidents vasculaires cérébraux et les traumatismes crâniens et médullaires, soit de type chronique comme les maladies autoimmunes (sclérose en plaques), la maladie d'Alzheimer, la maladie de Parkinson et toute autre maladie dans laquelle l'administration de facteurs neurotrophes est censée avoir un effet thérapeutique.

D'autres essais, tenant compte de la présence des récepteurs benzodiazépiniques périphériques dans divers organes du corps, ont montré que les composés de l'invention peuvent être utilisés comme agents cardioprotecteurs, comme agents rénoprotecteurs, comme agents de traitement des tumeurs et cancers et comme agents de traitement des stress cutanés.

A cet effet les composés de formule générale (I) peuvent être présentés sous toutes formes galéniques, seuls ou associés avec d'autres composés neurotrophes, neuroprotecteurs ou immunomodulateurs, et avec des excipients appropriés pour l'administration entérale ou parentérale, par exemple sous forme de comprimés, dragées, gélules, capsules, solutions ou suspensions buvables ou injectables telles que sirops ou ampoules, timbres transdermiques, suppositoires, etc, dosés pour permettre une administration journalière de 1 à 1000 mg de substance active.

## Revendications

1. Composé répondant à la formule générale (I) 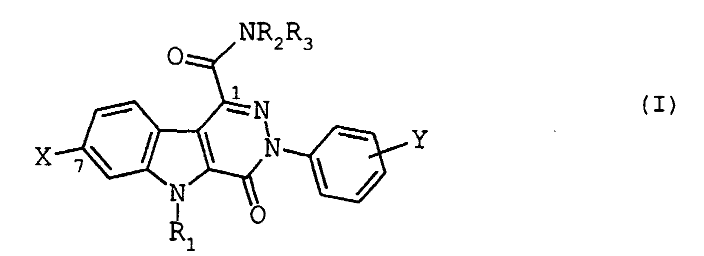 dans laquelle
X représente un atome d'hydrogène ou d'halogène,
Y représente un ou plusieurs atomes ou groupes choisis parmi l'hydrogène, les halogènes et les groupes méthyle, méthoxy et nitro,
R₁ représente un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle ou morpholinyle.

2. Composé selon la revendication 1, **caractérisé en ce que** X représente un atome d'halogène, Y représente un atome d'hydrogène ou un atome d'halogène, R₁ représente un groupe méthyle, R₂ représente un atome d'hydrogène ou un groupe méthyle, R₃ représente un groupe méthyle, ou bien R₂ et R₃ forment, avec l'atome d'azote qui les porte, un cycle pyrrolidinyle.

3. Médicament **caractérisé en ce qu'**il consiste en un composé selon l'une des revendications 1 et 2.

4. Composition pharmaceutique **caractérisée en ce qu'**elle contient un composé selon l'une des revendication 1 et 2, associé à un excipient.

## Patentansprüche

1. Verbindung der allgemeinen Formel (I) 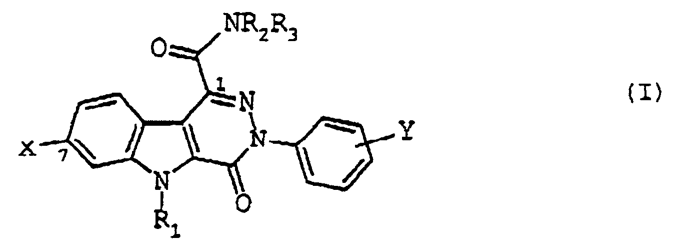 in der
X ein Wasserstoffatom oder ein Halogenatom,
Y ein oder mehrere Atome oder Gruppen ausgewählt aus Wasserstoff, Halogenen, Methyl-, Methoxy- und Nitrogruppen,
R₁ eine (C₁-C₄)-Alkylgruppe,
R₂ und R₃ jeweils unabhängig voneinander ein Wasserstoffatom oder eine (C₁-C₄)-Alkylgruppe bedeuten oder R₂ und R₃ zusammen mit dem sie tragenden Stickstoffatom eine Pyrrolidinyl-, Piperidinyl- oder Morpholinylgruppe bilden.

2. Verbindung nach Anspruch 1, **dadurch gekennzeichnet, daß** X ein Halogenatom, Y ein wasserstoffatom oder ein Halogenatom, R₁ eine Methylgruppe, R₂ ein Wasserstoffatom oder eine Methylgruppe, R₃ eine Methylgruppe oder R₂ und R₃ gemeinsam mit dem sie tragenden Stickstoffatom einen Pyrrolldinylring bedeuten.

3. Arzneimittel, **dadurch gekennzeichnet, daß** es aus einer Verbindung nach einem der Ansprüche 1 und 2 besteht.

4. Pharmazeutische Zubereitung, **dadurch gekennzeichnet, daß** sie eine Verbindung nach einem der Ansprüche 1 und 2 in Kombination mit einem Trägermaterial enthält.

## Claims

1. Compound corresponding to the general formula (I) 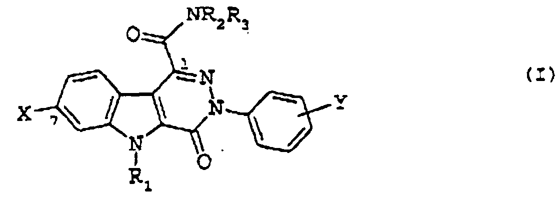 in which
X represents a hydrogen or halogen atom,
Y represents one or more atoms or groups chosen from hydrogen, halogens and methyl, methoxy and nitro groups,
R₁ represents a (C₁-C₄)alkyl group,
R₂ and R₃ each represent, independently of one another, a hydrogen atom or a (C₁-C₄)alkyl group or else R₂ and R₃ form, with the nitrogen atom which carries them, a pyrrolidinyl, piperidinyl or morpholinyl group.

2. Compound according to Claim 1, **characterized in that** X represents a halogen atom, Y represents a hydrogen atom or a halogen atom, R₁ represents a methyl group, R₂ represents a hydrogen atom or a methyl group and R₃ represents a methyl group or else R₂ and R₃ form, with the nitrogen atom which carries them, a pyrrolidinyl ring.

3. Medicament, **characterized in that** it comprises a compound according to either of Claims 1 and 2.

4. Pharmaceutical composition, **characterized in that** it comprises a compound according to either of Claims 1 and 2 in combination with an excipient.
